# EUROPEAN PATENT APPLICATION

(11) **EP 3 991 742 A1**
(43) Date of publication of application: **04.05.2022**
(21) Application number: 21751740.8
(22) Date of filing: 11.01.2021
(51) Int. Cl.: A61K 36/185, A61P 31/14, A23L 33/105

(54) **CORONAVIRUS THERAPEUTIC AGENT COMPRISING ELAEOCARPUS SYLVESTRIS EXTRACT AS ACTIVE INGREDIENT**

(30) Priority: 07.09.2020 KR 20200113730
(71) Applicant: Genencell Inc., Yongin-si, Gyeonggi-do 16950 (KR)
(72) Inventor: The designation of the inventor has not yet been filed
(74) Representative: Dehns
(86) International application number: PCT/KR2021/000347
(87) International publication number: WO 2022/050516

(57) **Abstract**

The present invention relates to a pharmaceutical composition for preventing, alleviating or treating coronavirus infection, containing an *Elaeocarpus sylvestris* leaf extract as an active ingredient, and a food composition for preventing or alleviating coronavirus infection. Since the *Elaeocarpus sylvestris* leaf extract exhibits excellent anti-viral activity against coronavirus which is a virus causing a respiratory disease, a digestive disease, a liver disease or a brain disease in a mammal, particularly, the corona-19 virus, it may be effectively used in medicines and food for preventing, alleviating or treating a disease caused by coronavirus infection.

## Description

### [Technical Field]

The present invention relates to a composition for preventing, alleviating or treating coronavirus infection, which includes an *Elaeocarpus sylvestris* extract.

### [Background Art]

Coronaviruses are RNA viruses causing respiratory diseases, digestive diseases, liver diseases and brain diseases in mammals and birds (Gallagher TM. et.al., Virology, 279(2): 371-374, 2001). Particularly, among viruses belonging to the coronaviruses, porcine transmissible gastroenteritis virus (TGEV) and porcine epidemic diarrhea virus (PEDV) are very highly infectious viruses which invade the digestive tract and digestive system, causing dehydration and high fever due to vomiting and diarrhea, and cause considerable economic loss due to a high mortality rate (Duarte M. et al., J Gen Virol., 75 (Pt 5):1195-1200, 1994). Although these viruses have a very high mortality rate, no therapeutic agent has been developed like for diseases caused by other viral infections. Therefore, vaccine development research for preventing viral infection is progressing, but its effectiveness is still low (Alonso S. et al., J Gen Virol., 83(Pt 3):567-579, 2002).

Coronavirus disease 2019 (COVID-19) or corona-19 caused by coronavirus is a respiratory infection caused by a new type of coronavirus (SARS-CoV-2; RNA virus belonging to Coronaviridae) that has spread across China and around the world since it first occurred in Wuhan, China in December 2019. While first known only as a respiratory infectious disease of unknown cause, the World Health Organization (WHO) revealed on January 9, 2020 that the cause of the pneumonia was a new type of coronavirus (SARS-CoV-2, named by the International Virus Classification Committee on February 11), confirmed as a pathogen.

The pathogen of COVID-19 is "SARS-coronavirus-2 (SARS-CoV-2)." The International Virus Classification Committee (ICTV) published a paper proposing the name SARS-CoV-2 as the pathogen of COVID-19 on February 11, 2020, and emphasized that the virus is similar to severe acute respiratory syndrome (SARS), which was prevalent in 2003.

The Korean Centers for Disease Control and Prevention obtained and analyzed the gene sequence of the virus released by China through academia, and confirmed that it has the highest homology (89.1%) with a bat-derived similar coronavirus.. It was confirmed that the homology with four types of human coronavirus is low as 39 to 43%, 50% with MERS, and 77.5% with SARS.

COVID-19, which is currently a global problem, is defined as a respiratory syndrome by novel coronavirus (severe acute respiratory syndrome coronavirus 2, SARS-CoV-2) infection, and classified into the category of statutory infectious diseases, first-degree infectious diseases, and new infectious disease syndromes, and the disease code for this is U07.1. The pathogen is SARS-CoV-2, which is an RNA virus belonging to Coronaviridae, and its route of transmission is known to be through droplets (saliva) and contact. Therefore, it is known that the virus is transmitted via droplets generated by coughing or sneezing or by touching objects contaminated with coronavirus and then touching one's eyes, nose and mouth.

The global fatality rate of coronavirus infection is known to be approximately 3.4% (3.5, based on the WHO) on average. However, the levels of fatality by country and age vary such that 6% of infected people are registered as dead worldwide, and the infection causes severe symptoms and death mainly in the elderly, immunocompromised patients and patients with underlying diseases.

Coronavirus is an RNA virus having a helical structure with a single positive strand and consists of a total of five structural proteins, and among these proteins, due to a spike glycoprotein (S protein), it is observed as appearing to have a crown or halo under electron microscopy (Schoeman and Fielding, 2019). Coronavirus is known to be the largest among the existing RNA viruses of 27 to 34 KB, and subclassified into four groups: alpha, beta, delta and gamma. Among these subgroups, the beta group is known to be highly contagious and virulent. SARS-CoV-2 invades while the S protein present on its surface binds to angiotensin converting enzyme 2 (ACE2) of host cells, and since ACE2 is widely distributed in the mucosa in the oral and nasal cavity, nasopharynx, lungs, stomach, small intestine and large intestine, viral pneumonia is commonly observed (Hamming et al., 2004).

The incubation period of coronavirus is 1 to 14 days (average 4 to 7 days), and as a diagnostic standard, a virus is isolated from a specimen from a human who has been infected with the pathogen of an infectious disease or a specific gene is detected from the specimen, and then used in diagnosis. Symptoms of COVID-19 include mild to severe various respiratory infection symptoms such as fever, malaise, coughing, breathing difficulty and pneumonia, and also phlegm, sore throat, headache, hemoptysis, nausea and diarrhea. Currently, there is no specific antiviral agent, other than conservative treatment such as fluid supplementation and an antipyretic drug, and the World Health Organization (WHO) announced that there is no evidence that people can be protected from reinfection and infection with COVID-19 even if there is an antibody against SARS-CoV-2, magnifying the importance of the development of a therapeutic agent.

*Elaeocarpus sylvestris* is a evergreen tall tree belonging to the family Elaeocarpaceae, and found in subtropical regions including Jeju Island in Korea, and the southern areas of China and Japan. *Elaeocarpus sylvestris* may be a resource that has little known active ingredients and efficacy, and a material with very high material differentiation and rarity of research and development.

However, no literature published to date disclosed that an *Elaeocarpus sylvestris* extract is effective in preventing, alleviating or treating coronavirus infection, particularly, COVID-19.

### [Related Art Document]

Korean Registered Patent No. 10-1834872

### [Disclosure]

### [Technical Problem]

The present invention is directed to providing a pharmaceutical composition for preventing, alleviating or treating coronavirus infection, which includes an *Elaeocarpus sylvestris* extract as an active ingredient.

The present invention is also directed to providing a food composition for preventing or alleviating coronavirus infection, which includes an *Elaeocarpus sylvestris* extract as an active ingredient.

The purpose of the present invention is not limited to the above-described objects. The purpose of the present invention will become clearer from the following description, and will be realized by means described in the claims and a combination thereof.

### [Technical Solution]

To achieve the above-described objects, the following solutions will be provided.

One aspect of the present invention provides a pharmaceutical composition for preventing, alleviating or treating coronavirus infection, which includes an *Elaeocarpus sylvestris* extract as an active ingredient.

In one embodiment of the present invention, the extract may be an extract of water, a C1 to C5 lower alcohol, or a C1 to C5 lower alcohol aqueous solution.

In another embodiment of the present invention, the extract may be extracted with a 30 to 95% ethanol aqueous solution.

In still another embodiment of the present invention, the extract may be extracted with a 40 to 60% ethanol aqueous solution.

In yet another embodiment of the present invention, the *Elaeocarpus sylvestris* extract may be an *Elaeocarpus sylvestris* leaf extract.

In yet another embodiment of the present invention, the coronavirus may be one or more selected from the group consisting of porcine transmissible gastroenteritis virus (TGEV), porcine epidemic diarrhea virus (PEDV), canine coronavirus, bovine coronavirus, SARS coronavirus (SARS-CoV) and corona-19 virus (SARS-CoV-2).

In yet another embodiment of the present invention, the pharmaceutical composition may include a pharmaceutically acceptable carrier, excipient or diluent.

In yet another embodiment of the present invention, the pharmaceutical composition may be orally administered in the form of one selected from the group consisting of a powder, granules, a tablet, a capsule and a liquid.

Another aspect of the present invention provides a food composition for preventing or alleviating coronavirus infection, which includes an *Elaeocarpus sylvestris* extract as an active ingredient.

### [Advantageous Effects]

An *Elaeocarpus sylvestris* extract of the present invention can exhibit excellent antiviral activity against coronaviruses which is a virus causing respiratory diseases, digestive diseases, liver diseases and brain diseases in mammals, particularly, the COVID-19 virus, and thus can be effectively used in pharmaceuticals and food for preventing, alleviating or treating a disease caused by coronavirus infection.

### [Description of Drawings]

FIG. 1 shows the coronavirus infection inhibitory effect of an *Elaeocarpus sylvestris* extract of the present invention in Vero-E6 host cells.
FIG. 2 shows the viral replication inhibitory effect of an *Elaeocarpus sylvestris* extract of the present invention in Vero-E6 host cells infected with coronavirus.
FIG. 3 shows the inhibitory effect on SARS-CoV-2 spike protein binding activity of an *Elaeocarpus sylvestris* extract of the present invention in SARS-CoV-2-infected hamsters.

### [Modes of the Invention]

Unless specified otherwise, since all numbers, values and/or expressions expressing components, reaction conditions and the content of an ingredient, which are used herein, are approximate values that reflect various uncertainties of measurement occurring to obtain these values among essentially different things, in all cases, it should be understood that the values are modified by the term "about". In addition, when a numerical range is specified in the specification, such a range is continuous, and unless indicated otherwise, includes all values from the minimum value to the maximum value of the range. Furthermore, when such a range indicates an integer, unless indicated otherwise, the range includes all integers between the minimum value and the maximum value.

In the specification, when the range is specified by variables, it will be understood that the variables include all values within the disclosed range including the end points disclosed in this range. For example, the range from "5 to 10" includes not only 5, 6, 7, 8, 9 and 10, but also any subrange such as 6 to 10, 7 to 10, 6 to 9 or 7 to 9, and it should be understood that any values between suitable integers in the range disclosed herein, such as 5.5, 6.5, 7.5, 5.5 to 8.5 and 6.5 to 9, are also included. In addition, for example, the range from "10 to 30%" includes not only all integers including 10%, 11%, 12%, 13%, and up to 30%, but also any subrange including 10 to 15%, 12 to 18% or 20 to 30%, and it should be understood that the range also includes any value between suitable integers in the range disclosed herein, such as 10.5%, 15.5% or 25.5%.

In addition, the terms and abbreviations used herein will be interpreted as meanings conventionally understood by one of ordinary skill in the art to which the present invention belongs.

The present invention relates to a composition for preventing, alleviating or treating coronavirus infection, which includes an *Elaeocarpus sylvestris* extract as an active ingredient, as a result of experiments for inhibiting SARS-CoV-2 virus infection and replication in Vero-E6 cells using the *Elaeocarpus sylvestris* extract, significant results were confirmed in a dose-dependent manner. In addition, as a result of confirming SARS-CoV-2 spike protein binding activity in coronavirus-infected hamster models, it was confirmed that the activity was reduced very well, and the inhibition of a body temperature increase and body weight loss in experimental animals was confirmed. Therefore, it was shown that the *Elaeocarpus sylvestris* extract inhibits the coronavirus infection of cells by suppressing the spike protein binding activity of SARS-CoV-2. According to the present invention, it will be possible to develop a composition for preventing or alleviating coronavirus infection, which has no toxicity at high concentrations and an excellent therapeutic effect, and it will be possible to develop it as a health functional food or pharmaceutical through additional research.

The present invention provides a pharmaceutical composition for preventing, alleviating or treating coronavirus infection, which includes an *Elaeocarpus sylvestris* extract as an active ingredient.

In one embodiment of the present invention, the "coronavirus" may be "beta coronavirus", but the present invention is not limited thereto.

In one embodiment of the present invention, the "coronavirus" may be "SARS-CoV-2 (COVID-19)", but the present invention is not limited thereto.

The present invention provides a health functional food for preventing or alleviating coronavirus infection, which includes an *Elaeocarpus sylvestris* extract as an active ingredient.

The present invention provides a food composition for preventing or alleviating coronavirus infection, which includes an *Elaeocarpus sylvestris* extract as an active ingredient.

There have been many infections and deaths worldwide due to various types of coronaviruses including SARS prevalent in 2002, MERS prevalent in 2012, and SARS-CoV-2 (COVID-19) being prevalent from the end of 2019 to 2020. Since coronavirus is an RNA virus and is easily mutated due to its nature, it has a characteristic that the probability of occurrence of a modified form of the virus is high. The *Elaeocarpus sylvestris* extract of the present invention exhibited a result of inhibiting viral infection and replication in a dose-dependent manner when Vero-E6 cells were infected with the SARS-CoV-2 virus, and suppressed a body temperature increase and a body weight loss in SARS-CoV-2-infected hamsters. In addition, the *Elaeocarpus sylvestris* extract exhibited a result of significantly inhibiting SARS-CoV-2 spike protein binding activity in SARS-CoV-2-infected hamsters. Therefore, the effect of inhibiting coronavirus replication and infection was confirmed by inhibiting the spike protein binding activity of SARS-CoV-2 using the *Elaeocarpus sylvestris* extract, and thus the present invention was completed.

Hereinafter, the present invention will be described in detail.

The present invention is directed to providing a method of extracting an *Elaeocarpus sylvestris* extract used in a composition for preventing or treating coronavirus infection, which includes an *Elaeocarpus sylvestris* extract as an active ingredient.

In the present invention, the extract is obtained by extraction and isolation from a natural substance using extraction and isolation methods known in the art, and the "extract" defined herein is extracted from *Elaeocarpus sylvestris* using a suitable solvent. The "extract" includes all of a crude extract, a polar solvent-soluble extract or a non-polar solvent-soluble extract. As a suitable solvent for extracting an extract from *Elaeocarpus sylvestris,* any one of pharmaceutically acceptable organic solvents can be used. As a solvent, various solvents, for example, distilled water, C1 to C4 alcohols such as ethanol, methanol, isopropanol and butanol, acetone, ether, benzene, chloroform, ethyl acetate, methylene chloride, hexane and cyclohexane may be used alone or in combination. An extraction method may be selected from heat extraction, cold extraction, reflux cooling extraction, steam distillation, ultrasonic extraction, elution and compression. In addition, depending on the purpose, the extract may be additionally subjected to a conventional fractionation process, and may be purified according to a conventional purification method.

There is no limitation to the method of preparing an extract of the present invention, and any method disclosed herein can be used. For example, the extract included in the composition of the present invention may be prepared by powdering a primary extract extracted by the above-listed hot water extraction or solvent extraction through an additional process such as distillation under reduced pressure and freeze drying or spray drying. In addition, a purified fraction may be additionally obtained by subjecting the primary extract to various chromatographic methods such as silica gel column chromatography, high performance liquid chromatography, and thin layer chromatography. Therefore, in the present invention, the extract may be understood as a concept encompassing all of extracts, isolated compounds, fractions and purified products obtained in each step of extraction, fractionation or purification, dilutions, concentrates and dry products thereof.

The present invention is directed to providing a pharmaceutical composition for preventing or treating coronavirus infection, which includes an *Elaeocarpus sylvestris* extract as an active ingredient.

The "treatment" used herein refers to all actions involved in alleviating the symptoms of coronavirus infection or changing the symptoms of coronavirus infection to a normal condition by the administration of the composition of the present invention. It is judged that one of ordinary skill in the art to which the present invention pertains would know the exact criteria for a therapeutic effect with reference to data presented by the Korean Medical Association, and the criteria may determine the degrees of alleviation, improvement and treatment of the symptoms of coronavirus infection with the composition of the present invention.

The pharmaceutical composition may be one or more selected from oral formulations, preparations for external use, suppositories, sterilized injectable solutions and sprays, and a sterilized injectable solution is preferably used.

The "prevention" used herein refers to all actions of inhibiting or delaying the occurrence, spread and recurrence of coronavirus infection by administration of the pharmaceutical composition according to the present invention.

The "therapeutically effective amount" used herein refers to the amount of a pharmaceutically acceptable salt as the amount of a composition effective for preventing or treating a disease caused by coronavirus infection. The therapeutically effective amount of the composition of the present invention may vary according to various factors including an administration method, a targeted site, and a patient's condition. Therefore, when the composition is applied to the human body, it should be administered at a proper amount in consideration of safety and efficiency, and an amount used in a human may be estimated from an effective amount determined through an animal experiment.

The "pharmaceutically effective amount" used herein refers to an amount sufficient for treating a disease at a reasonable benefit/risk ratio applicable for medical treatment and not causing side effects. An effective dosage may be determined according to parameters including a patient's health condition, the type of disease, the severity of the disease, drug efficacy, sensitivity to a drug, administration method, time and route, an excretion rate, the duration of treatment and drugs that are mixed or simultaneously used, and other parameters well known in the medical field. The composition of the present invention may be administered separately or in combination with other therapeutic agents, and may be sequentially or simultaneously administered with another therapeutic agent, or administered in a single or multiple dose(s). In consideration of all of the above-mentioned parameters, it is important to administer the composition of the present invention at an amount that can achieve the maximum effect with the minimum amount without a side effect, and such an amount is determined by one of ordinary skill in the art.

The pharmaceutical composition of the present invention may further include a pharmaceutically acceptable additive. The pharmaceutically acceptable additive may be microcrystalline cellulose, powdered cellulose, hydroxypropyl cellulose, starch, gelatinized starch, pregelatinized starch, corn starch, white sugar, lactose, dextrose, sorbitol, mannitol, malt, lactose, colloidal silicon dioxide, povidone, Opadry, synthetic aluminum silicate, stearic acid, magnesium stearate, aluminum stearate, calcium stearate or talc. The amount of the pharmaceutically acceptable additive for the composition of the present invention may be 0.1 to 90 parts by weight with respect to the composition, but the present invention is not limited thereto.

The pharmaceutical composition of the present invention may include a carrier, a diluent or an excipient, which is conventionally used in a biological preparation, or a combination thereof. The pharmaceutically acceptable carrier is not particularly limited as long as it is suitable for in vivo delivery of the composition. For example, the pharmaceutically acceptable carrier includes saline, sterilized water, Ringer's solution, a dextrose solution, a maltodextrin solution, glycerol, ethanol and a compound, which are disclosed in Merck Index, 13rd ed., Merck & Co. Inc. In addition, one or more components of these components may be mixed before use, and other conventional additives such as an antioxidant, a bacteriostatic agent and a buffer may be applied as needed. In addition, the pharmaceutical composition of the present invention may be prepared as an injectable formulation such as an aqueous solution, a suspension or an emulsion, a pill, a capsule, granules or a tablet by additionally adding a diluent, a surfactant, a dispersing agent, a binder or a lubricant. The pharmaceutical composition of the present invention is preferably prepared according to a component, a patient's disease and a severity using a method presented in Remington's Pharmaceutical Science (Mack Publishing Company, Easton PA, 18th, 1990) as a proper method in corresponding art.

The pharmaceutical composition of the present invention may be administered parenterally (intravenously, subcutaneously, intraperitoneally, or with a local injectable formulation) or orally. The dosage may vary depending on a patient's agent, sex, health condition, a body weight, a diet, an administration time and method, an excretion rate and the severity of a disease. A daily dose of the composition of the present invention may be 0.0001 to 10 mg/mL, and preferably, 0.0001 to 5 mg/mL, which may be administered once or in divided portions.

As a liquid formulation for oral administration, a suspension, a liquid for internal use, an emulsion, or a syrup may be used. A generally-used simple diluent such as sterilized water, saline or liquid paraffin, as well as various types of excipients such as a sweetener, a fragrance and a preservative and a wetting agent may be included. A formulation for parenteral administration may be a lyophilizing agent, a sterilized aqueous solution, a non-aqueous solvent, an emulsion, a suspension or a suppository.

The pharmaceutical composition of the present invention may be administered with a conventional composition for treating coronavirus infection to increase a coronavirus infection treatment effect, and concomitant administration may be performed simultaneously or sequentially with a conventional therapeutic composition.

The present invention is directed to providing a health functional food composition for preventing or alleviating coronavirus infection, which includes an *Elaeocarpus sylvestris* extract as an active ingredient.

When the *Elaeocarpus sylvestris* extract of the present invention is used in the health functional food composition, it may be used along with other foods or food ingredients, and may be appropriately used according to a conventional method.

The usage of the composition may include a sitologically acceptable food supplement in addition to the active ingredient, and the mixed amount of the active ingredient is properly determined according to the purpose of use (prevention or auxiliary treatment).

The "food supplement" used herein refers to a constituent that can be supplementally added to food. This is an element added to prepare each formulation of health functional food, and may be suitably selected by those of ordinary skill in the art. Examples of the food supplements include various nutrients, vitamins, electrolytes, synthetic and natural flavors, coloring agents, fillers, pectic acid and a salt thereof, alginic acid and a salt thereof, organic acids, protective colloidal thickening agents, pH adjustors, stabilizers, preservatives, glycerin, alcohols, or carbonating agents, but the present invention is not limited thereto.

In addition, the composition of the present invention may be applied to the health functional food. The "health functional food" used herein refers to food produced and processed in the form of a tablet, a capsule, granules, a liquid, a pill or a powder using a raw material or component with useful functionality for the human body. The "functionality" refers to obtaining an effect useful for health, such as controlling nutrients or physiological actions for the human body.

The composition of the present invention may be prepared in various forms as a food composition, and is prepared in any form recognized as health functional food without limitation. When ingested as a food composition, the composition has an advantage of no side effects that can occur in long-term use of a medicine.

The composition of the present invention can be prepared as a health functional food by a method conventionally used in the related art, and may be prepared by adding raw materials and components, which are conventionally used in preparation. In addition, the health functional food prepared using the composition can be applied without limitation as long as it can be prepared in the form of a health functional food.

Since there is no limitation on the type of health functional food for which the composition of the present invention can be used, the composition including the *Elaeocarpus sylvestris* extract of the present invention as an active ingredient may be prepared by mixing suitable auxiliary components and additives, which may be contained in the health functional food according to the choice of one of ordinary skill in the art. Examples of the food to which the *Elaeocarpus sylvestris* extract can be added include meat, sausage, chocolate, candy, snacks, confectioneries, bread, ramen and other noodles, gum, dairy products including ice cream, various types of tea, beverages, drinks, alcoholic beverages and vitamin complexes. In addition, the health functional food may be prepared by adding the *Elaeocarpus sylvestris* extract according to the present invention as a main ingredient to an extract, tea or juice.

Hereinafter, various aspects of the present invention will be described.

One aspect of the present invention provides a pharmaceutical composition for preventing, alleviating or treating coronavirus infection, which includes an *Elaeocarpus sylvestris* extract as an active ingredient.

Another aspect of the present invention provides a method of preventing, alleviating or treating coronavirus infection, which includes administering an effective amount of the *Elaeocarpus sylvestris* extract to a subject in need of prevention, alleviation or treatment of coronavirus infection.

Still another aspect of the present invention provides an *Elaeocarpus sylvestris* extract to be used in preventing, alleviating or treating coronavirus infection.

Yet another aspect of the present invention provides a use of an *Elaeocarpus sylvestris* extract for the manufacture of a medicament for preventing, alleviating or treating coronavirus infection.

In one embodiment of the present invention, the extract is an extract of water, a C1 to C5 lower alcohol, or a C1 to C5 lower alcohol aqueous solution.

In one embodiment of the present invention, the extract is extracted with an 30 to 95% ethanol aqueous solution.

In one embodiment of the present invention, the extract is extracted with a 40 to 60% ethanol aqueous solution.

In one embodiment of the present invention, the *Elaeocarpus sylvestris* extract is an *Elaeocarpus sylvestris* leaf extract.

In one embodiment of the present invention, *Elaeocarpus sylvestris* leaves may be cultivated or purchased without limitation. In addition, in addition to the *Elaeocarpus sylvestris* leaves, the whole plant, including stems, branches and roots, may be extracted.

In one embodiment of the present invention, it is preferable to use water, an alcohol or a mixture thereof as the extraction solvent. The alcohol is preferably a C₁ to C₄ lower alcohol, and the lower alcohol is preferably ethanol. The ethanol is, but not limited to, preferably a 30 to 95% ethanol aqueous solution, and more preferably a 45 to 55% ethanol aqueous solution. Aa an extraction method, shaking extraction, Soxhlet extraction or reflux extraction is preferably used, but the present invention is not limited thereto. The extraction is preferably performed by adding the extraction solvent in an amount of 1 to 30-fold, 1 to 20-fold, or 1 to 10-fold the amount of dried *Elaeocarpus sylvestris* leaves. An extraction temperature is preferably 20 to 100 °C, more preferably 20 to 80 °C, and most preferably room temperature, but the present invention is not limited thereto. In addition, the extraction time is preferably 1 to 10 days, but the present invention is not limited thereto. In addition, the number of extractions may be one or more, but as the extraction continues, the yield of an active ingredient is remarkably reduced, and therefore it may not be economical to repeat more than 5 times. Accordingly, the number of extractions is preferably 1 to 5 times, and more preferably 2 to 5 times, but the present invention is not limited thereto.

The extraction method is a method conventionally known in the art, and for example, a method using an extraction apparatus, such as supercritical extraction, subcritical extraction, high temperature extraction, high pressure extraction or ultrasonic extraction, or a method using an adsorption resin including XAD and HP-20 may be used.

In this method, vacuum evaporation preferably uses a vacuum evaporator or rotary evaporator, but the present invention is not limited thereto. In addition, drying is preferably decompression drying, vacuum drying, boil drying, spray drying or freeze drying, but the present invention is not limited thereto.

In one embodiment of the present invention, the coronavirus is one or more selected from the group consisting of porcine transmissible gastroenteritis virus (TGEV), porcine epidemic diarrhea virus (PEDV), canine coronavirus, bovine coronavirus, SARS coronavirus (SARS-CoV) and corona-19 virus (SARS-CoV-2).

In one embodiment of the present invention, the coronavirus is beta coronavirus.

In one embodiment of the present invention, the pharmaceutical composition includes a pharmaceutically acceptable carrier, excipient or diluent.

In one embodiment of the present invention, the pharmaceutical composition is prepared in the form of one selected from the group consisting of a powder, granules, a tablet, a capsule and a liquid for oral administration.

The pharmaceutical composition according to the present invention includes an *Elaeocarpus sylvestris* leaf extract as an active ingredient, and the active ingredient may be included at 0.1 to 50 wt% with respect to the total weight of the composition, but the present invention is not limited thereto.

Meanwhile, the pharmaceutical composition of the present invention is administered at a pharmaceutically effective amount. The term "administration" used herein refers to the introduction of a predetermined material into a subject, and the administration route of the composition may be any common route that can reach target tissue. The administration route may be intraperitoneal administration, intravenous administration, intramuscular administration, subcutaneous administration, intradermal administration, oral administration, local administration, intranasal administration, intrapulmonary administration or intrarectal administration, but the present invention is not limited thereto.

The term "subject" refers to all animals including a human, a rat, a mouse and livestock. Preferably, the subject includes mammals including a human.

The "pharmaceutically effective amount" used herein refers to an amount sufficient for treating a disease at a reasonable benefit/risk ratio applicable for medical treatment and not causing side effects, and an effective dosage may be easily determined by those of ordinary skill in the art according to parameters including a patient's sex, age, body weight and health condition, the type of disease, the severity of the disease, drug efficacy, sensitivity to a drug, administration method, time and route, an excretion rate, the duration of treatment and drugs that are mixed or simultaneously used, and other parameters well known in the medical field. However, for a preferable effect, in the case of an adult, the composition of the present invention may be included at a dose of 0.0001 to 1,000 mg/kg, and preferably, 10 to 300 mg/kg (body weight). The daily dose may be administered once or in divided portions. The dose may not limit the scope of the present invention in any way.

Yet another aspect of the present invention provides a food composition for preventing or alleviating coronavirus infection, which includes an *Elaeocarpus sylvestris* extract as an active ingredient.

The effect of the *Elaeocarpus sylvestris* extract in preventing, alleviating or treating coronavirus infection is as described above, and when the composition of the present invention is used as a food additive, the *Elaeocarpus sylvestris* extract may be used alone or used in combination with other foods or food ingredients, and may be appropriately used according to a conventional method. The mixing amount of the active ingredient may be suitably determined depending on the purpose of use (prevention, health or therapeutic treatment), and may further include a sitologically acceptable food supplement. Since the composition of the present invention includes an extract derived from a natural substance and a fraction thereof as an active ingredient, there is no problem in terms of stability, so there is no great limitation on a mixing amount.

The food composition of the present invention may include all types of food in the general sense, and is interchangeably used with terms known in the art, such as functional food, health functional food or the like.

The term "functional food" used herein refers to food that is produced and processed using raw materials or ingredients having functionality useful for the human body according to the Korean Act No. 6727 on Health Functional Food, and the "functionality" means ingestion for the purpose of regulating nutrients in terms of the structure and function of the human body or obtaining an effect useful for health, such as a physiological action.

In addition, the term "health functional food" used herein refers to food that is produced and processed through extraction, concentration, purification or mixing of a specific component used as a raw material or contained in a food ingredient for the purpose of health supplementation, and food that is designed and processed to sufficiently exhibit bioregulatory functions such as bio-defense, the control of biorhythms, and the prevention and recovery of a disease in the human body by the ingredient. The composition for health food may perform functions associated with the prevention and recovery of a disease.

There is no limitation on the type of food containing the composition of the present invention. In addition, the composition of the present invention including the *Elaeocarpus sylvestris* extract as an active ingredient may be prepared by mixing other suitable auxiliary ingredients and known additives, which can be contained in food by the choice of one of ordinary skill in the art. Examples of food to which the *Elaeocarpus sylvestris* extract can be added include meat, sausage, bread, chocolate, candy, snacks, confectioneries, pizza, ramen, other noodles, gum, dairy products including ice cream, various types of soups, beverages, tea, drinks, alcoholic beverages and vitamin complexes, and the health functional food may be prepared by adding the extract according to the present invention and a fraction thereof as a main ingredient to an extract, a tea, a jelly and a juice.

In addition, the food that can be applied to the present invention may include all types of food, for example, special nutritional food (e.g., milk formula, infant/baby food, etc.), processed meat products, fish meat products, tofu, acorn jelly, noodles (e.g., ramen, noodle etc.), health supplement food, seasonings (e.g., soy source, bean paste, red pepper paste, mixed paste, etc.), sauces, confectioneries (e.g., snacks), dairy products (e.g., fermented milk, cheese, etc.), other processed food, kimchi, pickled food (various types of kimchi, pickles, etc.), beverages (e.g., fruit, vegetable, soy milk, fermented beverages, etc.), and natural seasonings (e.g., ramen powder, etc.).

When the health functional food composition of the present invention is used in the form of a beverage, it may contain various sweetening agents, flavoring agents or natural carbohydrates as additional ingredients like general beverages. In addition, the composition of the present invention may contain various nutrients, vitamins, electrolytes, flavoring agents, pectic acid and a salt thereof, alginic acid and a salt thereof, organic acids, protective colloidal thickening agents, pH adjustors, stabilizers, preservatives, glycerin, alcohols, or carbonizing agents. Other than these, the composition of the present invention may contain fruit pulp for producing natural fruit juices, fruit drinks and vegetable drinks.

### [Examples]

Hereinafter, the configuration and effects of the present invention will be described in further detail with respect to examples, experimental examples and preparation examples. These examples, experimental examples and preparation examples are merely provided to illustrate the present invention, but the scope of the present invention is not limited by the examples.

### Example 1. Preparation of Elaeocarpus sylvestris extract

After *Elaeocarpus sylvestris* leaves native to Jeju Island, Korea were purchased, dried and chopped, 100 kg of the *Elaeocarpus sylvestris* leaves was extracted with 2,000 L of 50% ethanol at 60 °C for 16 hours, filtered, concentrated and then dried, thereby obtaining 28 kg of an *Elaeocarpus sylvestris* extract (yield: 28%).

### Experimental Example 1. Viral infection inhibitory effect of Elaeocarpus sylvestris extract

Vero-E6 cells were cultured in DMEM (-/-), and inoculated into a 12 well-plate at concentration of 5×10⁵ cells/well in a viral infection experiment. The cells were incubated at 37 °C in a CO₂ incubator for 24 hours, and washed twice with 1× PBS following removal of the cell medium. The *Elaeocarpus sylvestris* extract was pretreated by concentration (25, 50 or 100 µg/mL) and incubated at 37 °C in a CO₂ incubator for 2 hours. Subsequently, for viral infection, the cell medium was removed, and the SARS-CoV-2 virus was treated at 50 pfu/well for 1 hour. Afterward, the cell medium was removed, 1.5 mL of the medium was added to each well and reacted for 72 hours, and then the result was confirmed. Here, 5 µM remdesivir (Rem) known to be effective in treating SARS-CoV-2 virus and 10 µMof chloroquinone phosphate (C.P), which is a therapeutic agent for malaria, were set as comparative groups to carry out the experiment.

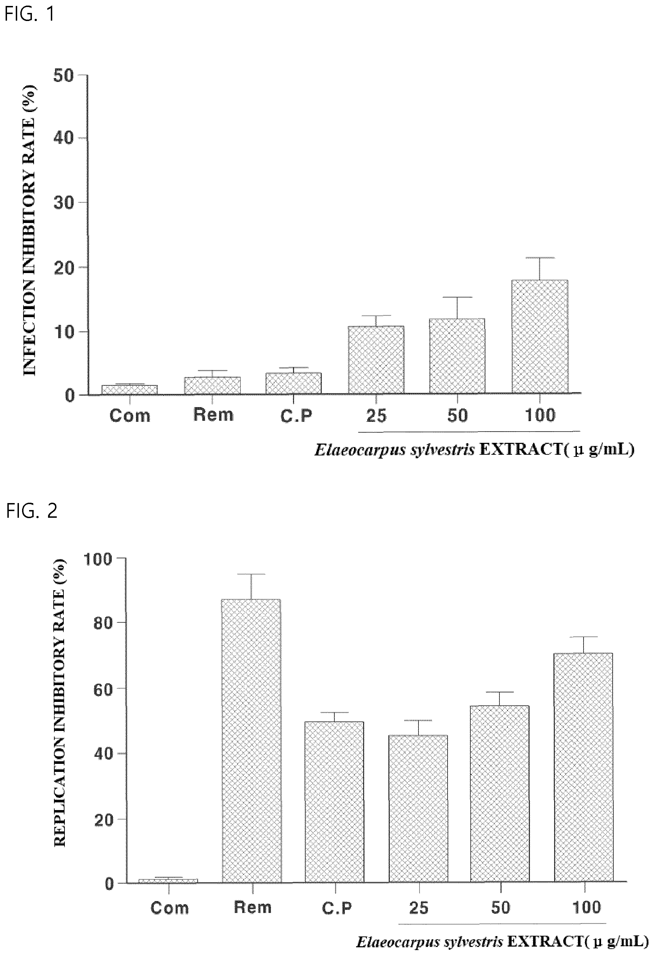

As shown in FIG. 1, the *Elaeocarpus sylvestris* extract exhibited an excellent virus infection inhibitory effect, and the result is achieved in a dose-dependent manner. Compared to the comparative groups, Rem or C.P, the *Elaeocarpus sylvestris* extract exhibited a much better result.

### Experimental Example 2. Viral replication inhibitory effect of Elaeocarpus sylvestris extract

Vero-E6 cells were cultured in DMEM (-/-), and inoculated into a 12 well-plate at concentration of 5×10⁵ cells/well. The cells were incubated at 37 °C in a CO₂ incubator for 24 hours, and then the cell medium was removed, followed by washing twice with 1× PBS. For viral infection, the SARS-CoV-2 virus was treated at 50 pfu/well for 1 hour, 1.5 mL of a medium containing the *Elaeocarpus sylvestris* extract by concentration (25, 50 or 100 µg/mL) was added to each well and reacted for 72 hours, and then the result was confirmed. Here, 5 µM Remdesivir (Rem) known to be effective in treating the SARS-CoV-2 virus and 10 µM of chloroquinone phosphate (C.P), which is a therapeutic agent for malaria, were set as comparative groups to carry out the experiment.

As shown in FIG. 2, the *Elaeocarpus sylvestris* extract exhibited an effective SARS-CoV-2 virus replication inhibitory effect, and the result was achieved in a dose-dependent manner. The antiviral effect was exhibited without cytotoxicity even at a high concentration. Compared to the C.P result for the viral replication inhibitory effect, the result of the *Elaeocarpus sylvestris* extract was excellent, and from the concentration of 50 µg/mL or more, the result of the *Elaeocarpus sylvestris* extract was superior to that of C.P. However, all concentrations of the *Elaeocarpus sylvestris* extract showed slightly lower levels of replication inhibitory ability than Rem.

### Experimental Example 3. Antiviral effect of Elaeocarpus sylvestris extract

6-week-old hamsters were obtained, randomly divided into groups, and acclimated for 1 week. The hamsters were infected with SARS-CoV-2, and after 24 hours, the *Elaeocarpus sylvestris* extract was orally administered at a dose of 25, 50 or 100 mg/kg for 4 days. Before and for 4 days after virus administration, the body weights of the hamsters were measured daily, and temperatures were measured once before and after infection.

On day 4 after virus administration, the hamster's saliva was harvested and pretreated, and then spike protein binding activity was confirmed using a SARS-CoV-2 Spike RBD Nanobody kit, thereby measuring an antiviral effect.

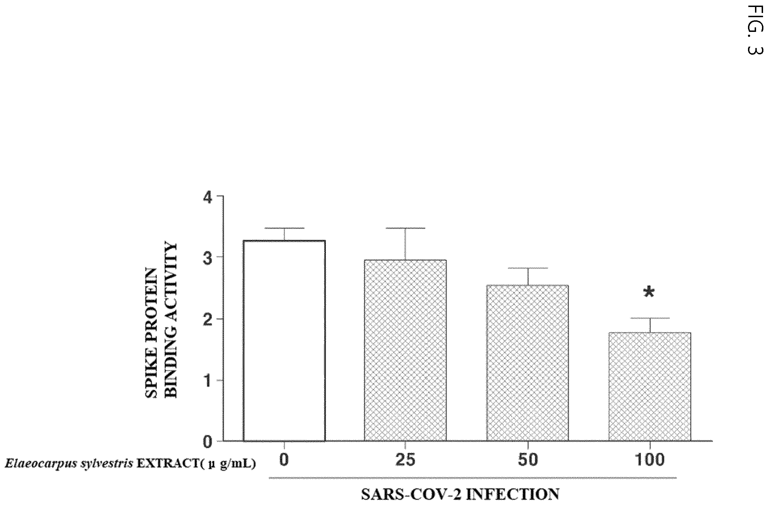

As a result, as shown in FIG. 3, the *Elaeocarpus sylvestris* extract exhibited a virus binding activity inhibitory effect, which was dose-dependent, and showed a statistically significant result (P<0.05) at a high concentration.

### Experimental Example 4. Antipyretic effect of Elaeocarpus sylvestris extract

COVID-19 is accompanied by fever due to viral infection, and therefore, in the experiment of Experimental Example 3, by measuring the body temperatures of hamsters on day 0 and day 4 of the infection, a COVID-19 symptom relief effect was measured.

As a result, as shown in Table 1, while the body temperature was increased by coronavirus infection, it was confirmed the increased body temperature was suppressed by the administration of the *Elaeocarpus sylvestris* extract, and its reduction effect was exhibited in a dose-dependent manner.

**[Table 1]**

| | Temperature (°C) | |
|---|---|---|
| | Infection period (day) | |
| | Day 0 (immediately before infection) | 4 days after infection |
| Control | 39.01 ± 1.766 | 41.33 ± 1.532 |
| *Elaeocarpus sylvestris* extract, 25 mg/kg | | 40.43 ± 1.942 |
| *Elaeocarpus sylvestris* extract, 50 mg/kg | | 39.70 ± 1.890 |
| *Elaeocarpus sylvestris* extract, 100 mg/kg | | 39.10 ± 1.900 |

Table 1. Increased temperature inhibitory effect of *Elaeocarpus sylvestris* extract in coronavirus-infected hamsters

### Experimental Example 5. Body weight loss inhibitory effect of Elaeocarpus sylvestris extract

COVID-19 causes a body weight decrease by viral infection, and in the experiment of Experimental Example 3, from day 0 to day 4 of the infection, the body weight of a hamster was measured a total of five times, thereby measuring a body weight loss inhibitory effect.

As a result, as shown in Table 2, while the body weight decreased by coronavirus infection, it was confirmed that a body weight loss was inhibited due to the administration of the *Elaeocarpus sylvestris* extract (ESE), and its reduction effect was exhibited in a dose-dependent manner.

**[Table 2]**

| Infection period (day) | Weight (g) | | | |
|---|---|---|---|---|
| | Virus infection: SARS-CoV-2 | | | |
| | *Elaeocarpus sylvestris* extract ( mg/kg) | | | |
| | 0 | 25 | 50 | 100 |
| 0 | 109.2 ± 2.87 | 109.6 ± 2.36 | 109.8 ± 2.69 | 109.9 ± 3.13 |
| 1 | 109.1 ± 2.91 | 109.3 ± 2.51 | 109.7 ± 3.14 | 109.7 ± 3.36 |
| 2 | 108.9 ± 2.82 | 109.3 ± 2.43 | 109.7 ± 2.88 | 109.7 ± 3.09 |
| 3 | 108.6 ± 2.63 | 109.2 ± 2.09 | 109.8 ± 3.08 | 109.7 ± 3.15 |
| 4 | 108.2 ± 2.56 | 109.0 ± 2.15 | 109.1 ± 2.59 | 109.6 ± 3.53 |

Table 2. Body weight loss inhibitory effect of *Elaeocarpus sylvestris* extract in coronavirus-infected hamsters

According to the experimental examples, the *Elaeocarpus sylvestris* extract of the present invention exhibited significant infection and replication inhibitory effect against the SARS-CoV-2 virus. In addition, after the hamster was infected with the virus, the body weight, the body temperature and the SARS-CoV-2 spike protein binding ability were measured, thereby confirming antiviral efficacy. Taking the experimental examples together, it is expected that the *Elaeocarpus sylvestris* extract inhibits coronavirus by the inhibition of RNA replication and the suppression of protein-binding ability of SARS-CoV-2. The *Elaeocarpus sylvestris* extract of the present invention can be developed as a composition for preventing, alleviating and treating coronavirus infection, which has no toxicity even at a high concentration and has an excellent therapeutic effect, and may have great potential for development as a food supplement or additive, health functional food or a medicine.

### [Preparation Examples]

Hereinafter, preparation examples for preparing a pharmaceutical composition or food composition, which includes the extract of Example 1 according to the present invention as an active ingredient, will be provided. The following preparation examples are merely provided to describe the present invention in detail, and the present invention is not limited thereto.

### Preparation Example 1: Preparation of pharmaceutical composition

### Preparation Example 1-1: Preparation of tablet

A binding liquid was prepared by dissolving 10 g of povidone in 50 g of ethanol, and 150 g of an active ingredient was added to the binding liquid and then uniformly stirred. After mixing 130 g of silicified microcrystalline cellulose, 15 g of croscarmellose sodium and 20 g of colloidal silicon dioxide, the binding liquid that had been uniformly stirred with the active ingredient was added to carry out a granulation process, followed by drying and granulation into granules. 15 g of crospovidone was added to the prepared granules and then mixed together, and 3.5 g of colloidal silicon dioxide and 6.5 g of magnesium stearate were added to carry out a lubrication process, followed by preparing a tablet.

### Preparation Example 1-2: Preparation of tablet

A binding liquid was prepared by dissolving 19 g of povidone in 50 g of ethanol, and 150 g of an active ingredient was added to the binding liquid and then uniformly stirred. After mixing 143 g of silicified microcrystalline cellulose, 15 g of croscarmellose sodium and 20 g of Neusilin, the binding liquid that had been uniformly stirred with the active ingredient was added to carry out a granulation process, followed by drying and granulation into granules. 15 g of crospovidone was added to the prepared granules and then mixed together, and 5 g of colloidal silicon dioxide and 8 g of magnesium stearate were added to carry out a lubrication process, followed by preparing a tablet.

### Preparation Example 1-3: Preparation of tablet

A binding liquid was prepared by dissolving 12.5 g of povidone in 50 g of ethanol, and 150 g of an active ingredient was added to the binding liquid and then uniformly stirred. After mixing 62.5 g of silicified microcrystalline cellulose, 65 g of lactose hydrate, 15 g of croscarmellose sodium and 20 g of colloidal silicon dioxide, the binding liquid that had been uniformly stirred with the active ingredient was added to carry out a granulation process, followed by drying and granulation into granules. 15 g of crospovidone was added to the prepared granules and then mixed together, and 3.5 g of colloidal silicon dioxide and 6.5 g of magnesium stearate were added to carry out a lubrication process, followed by preparing a tablet.

### Preparation Example 1-4: Preparation of tablet

A binding liquid was prepared by dissolving 19 g of povidone in 33.5 g of ethanol, and 150 g of an active ingredient was added to the binding liquid and then uniformly stirred. After mixing 105 g of silicified microcrystalline cellulose (the silicified microcrystalline cellulose was added in an amount as much as the amount of ethanol contained in the main component), 17.5 g of croscarmellose sodium, and 20 g of Neusilin, the binding liquid that had been uniformly stirred with the active ingredient was added to carry out a granulation process, followed by drying and granulation into granules. 17.5 g of crospovidone and 33 g of silicified microcrystalline cellulose were added to the prepared granules and mixed together, and 7.5 g of colloidal silicon dioxide and 8 g of magnesium stearate were added to carry out a lubrication process, followed by preparing a tablet.

### Preparation Example 1-5: Preparation of coated tablet

Coated tablets were prepared from 755 mg of the tablets (containing 300 mg of the active ingredient) prepared in each of Preparation Example 1-1 to 1-4 using a conventional pan coating system (Opadry^{®}, Opadry200^{®} or Opaglos^{®}).

### Preparation Example 1-6: Preparation of injectable solution

1 g of an active ingredient, 0.6 g of sodium chloride and 0.1 g of ascorbic acid were dissolved in distilled water, thereby preparing a 100 mL solution. This solution was poured in a bottle, and sterilized by heating it at 20 °C for 30 minutes.

### Preparation Example 2: Preparation of health food

1000 mg of an active ingredient, 1000 mg of citric acid, 100 g of oligosaccharide and 1 g of taurine were mixed, and distilled water was added to a final volume of 900 mL. After stirring and heating at 85 °C for approximately 1 hour, and the prepared solution was filtered and put in a sterilized 2 L container, sealed and sterilized, thereby preparing a beverage.

## Claims

1. A pharmaceutical composition for preventing, alleviating or treating coronavirus infection, comprising:
an *Elaeocarpus sylvestris* extract as an active ingredient.

2. The pharmaceutical composition of claim 1, wherein the extract is an extract of water, a C1 to C5 lower alcohol, or a C1 to C5 lower alcohol aqueous solution.

3. The pharmaceutical composition of claim 1, wherein the extract is extracted with a 30 to 95% ethanol aqueous solution.

4. The pharmaceutical composition of claim 1, wherein the extract is extracted with a 40 to 60% ethanol aqueous solution.

5. The pharmaceutical composition of claim 1, wherein the *Elaeocarpus sylvestris* extract is an *Elaeocarpus sylvestris* leaf extract.

6. The pharmaceutical composition of claim 1, wherein the coronavirus is one or more selected from the group consisting of porcine transmissible gastroenteritis virus (TGEV), porcine epidemic diarrhea virus (PEDV), canine coronavirus, bovine coronavirus, SARS coronavirus (SARS-CoV) and corona-19 virus (SARS-CoV-2).

7. The pharmaceutical composition of claim 1, wherein the pharmaceutical composition comprises a pharmaceutically acceptable carrier, excipient, or diluent.

8. The pharmaceutical composition of claim 1, wherein the pharmaceutical composition is selected from the group consisting of a powder, granules, a tablet, a capsule and a liquid for oral administration.

9. A food composition for preventing or alleviating coronavirus infection, comprising an *Elaeocarpus sylvestris* extract as an active ingredient.
